Europäisches Patentamt

European Patent Office

Office européen des brevets

(11) Veröffentlichungsnummer : **0 077 437 B1**

(12) **EUROPÄISCHE PATENTSCHRIFT**

(45) Veröffentlichungstag der Patentschrift :
13.03.85

(21) Anmeldenummer : 82106528.1

(22) Anmeldetag : 20.07.82

(51) Int. Cl.⁴ : **C 07 C 1/04, C 10 J 3/08, C 07 C 1/06**

(54) **Verfahren zur Herstellung benzinartiger Kohlenwasserstoffe aus Kohlegas.**

(30) Priorität : 20.10.81 DE 3141470
12.12.81 DE 3149273

(43) Veröffentlichungstag der Anmeldung :
27.04.83 Patentblatt 83/17

(45) Bekanntmachung des Hinweises auf die Patenterteilung : 13.03.85 Patentblatt 85/11

(84) Benannte Vertragsstaaten :
AT BE FR GB IT LU NL SE

(56) Entgegenhaltungen :
GB-A- 503 247
GB-A- 575 378
US-A- 2 583 164

(73) Patentinhaber : KLÖCKNER-WERKE AKTIENGESELL-SCHAFT
Klöcknerstrasse 29
D-4100 Duisburg 1 (DE)

(72) Erfinder : von Bogdandy, Ludwig, Dr. Ing. Prof.
Hirschkampstrasse 12
D-4200 Oberhausen-Sterkrade (DE)
Erfinder : Henkel, Siegfried, Dr. Ing.
An den Kiefern
D-2861 Garstedt (DE)

(74) Vertreter : Kador . Klunker . Schmitt-Nilson . Hirsch
Corneliusstrasse 15
D-8000 München 5 (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren zur Herstellung benzinartiger Kohlenwasserstoffe, bei dem ein Kohlegas, d. h. ein CO- und $H_2$-haltiges Gas, im erforderlichen Mengenverhältnis mit Wasser, insbesondere Wasserdampf gemischt wird und dieses Gemisch über einen Eisen-Katalysator geführt wird.

Bei diesem z. B. aus der Veröffentlichung in « Stahl und Eisen », 21.08.1958, S 1165 bekannten Verfahren zur Kohleverflüssigung (sogenanntes Kölbel-Engelhardt-Verfahren) wird Kohlenoxid (oder ein kohlenoxidhaltiges Gas wie Generatorgas, Gichtgas) mit Wasserdampf gemischt. Dieses Gemisch läßt man bei 180 bis 280 °C und normalem oder erhöhtem Druck über einen Eisen-Katalysator oder über einen anderen Katalysator, insbesondere ebenfalls aus der achten Gruppe des Periodensystems, streichen, wobei eine vollständige des Periodensystems, streichen, wobei eine vollständige Umsetzung zu Benzin, Paraffin und anderen Kohlenwasserstoffen erfolgt.

Die Verwendung von Gichtgas zur Synthese von Kohlenwasserstoffen hat sich bewährt, weil das Gichtgas nahezu schwefelfrei den Hochofen verläßt. Die für die Kohlenwasserstoffsynthese üblicherweise verwendeten Katalysatoren, auch das Eisen, sind schwefelempfindlich, sie werden durch Schwefel vergiftet. Bei Verwendung von Gichtgas ist, im Gegensatz zu den meisten anderen CO-haltigen Gasen, eine Nachentschwefelung nicht unbedingt notwendig.

Bei der praktischen Durchführung des bekannten Syntheseverfahrens wird das vom Staub befreite, maschinenreine Gichtgas auf etwa 15 bar verdichtet und zusammen mit Wasserdampf in einen Syntheseofen eingegeben. In diesem befindet sich der Eisenkatalysator entweder in Form gekörnter Teilchen und ist zwischen Kühlflächen fest angeordnet (sogenanntes Festbettverfahren) oder er ist fein vermahlen in Kohlenwasserstoffölen suspendiert. Dabei perlt das Gemisch aus Gichtgas und Wasserdampf durch diese Öl-Kontakt-Suspension (sogenannte Flüssigphase-Technik).

Die Flüssigphase-Technik hat wegen der Einfachheit der Geräte und der Wärmeabfuhr gegenüber der Synthese an fest angeordneten Katalysatoren bedeutende verfahrenstechnische Vorteile. Die Reaktion verläuft im Syntheseofen unter starker Wärmeentwicklung, es entstehen je Mol an —$CH_2$— etwa 58,4 kcal, also rund 780 kcal je umgesetztem $Nm^3$ Kohlenmonoxid. Diese Wärme wird durch Verdampfung von Wasser unter Druck in einem in den Syntheseofen eingebauten System von Rohrschlangen abgeführt.

Angesichts der bislang stets preiswerteren, aus Erdöl gewonnenen benzinartigen Kohlenwasserstoffe ist eine großtechnische Anwendung des bekannten Verfahrens bislang nicht wirtschaftlich gewesen. Insbesondere stören bei dem bevorzugt als CO- und $H_2$-haltiges Gas verwendeten Gichtgas die hohen Ballastanteile, die den gesamten Prozeß kostenmäßig stark belasten. Hierzu zählen die Verdichtung und Erwärmung des Gases, aber auch die notwendige Entstaubung sowie die Abkühlung.

Aufgabe der Erfindung ist es, Nachteile des bekannten Verfahrens zur Herstellung von benzinartigen Kohlenwasserstoffen zu vermeiden und ein Verfahren der eingangs genannten Art zu schaffen, bei dem energieaufwendige Schritte eingespart werden und zugleich ein normalerweise als Abfallprodukt anfallendes Material einer hochwertigen Weiterverarbeitung zugeführt werden kann.

Diese Aufgabe wird dadurch gelöst, daß als Eisenkatalysator die bei der Kohlevergasung im Eisenbadreaktor im erzeugten Kohlegas suspendierten, feinstverteilten Eisentropfen verwendet werden.

Die Kohlevergasung in einem Eisenbadreaktor ist beispielsweise aus der DE-OS 19 55 115 bekannt. Bei der Kohleveredlung im Eisenbadreaktor entsteht ein CO- und $H_2$-reiches Kohlegas. Dieses enthält jedoch, aufgrund der unvermeidlichen Eisenverdampfung im Reaktor, eine Suspension feinstverteilter Eisentropfen. Sie haben typischerweise einen Durchmesser unter 0,1 Mikrometer. Überschlägig fallen pro Tonne Kohle bei der Vergasung etwa 40 kg derartiger Eisentropfen an. Bei der bisherigen Verwendung des Kohlegases nach dem Stand der Technik, z. B. in Hüttenwerken, insbesondere in Walzwerksöfen, ist der hohe mitgeführte Gehalt an Eisentropfen störend und nachteilig. Die Eisentropfen lagern sich in den Leitungen ab, so daß sich die Leitungen zusetzen und stören an der Verbrennungsstelle.

Es hat demzufolge bislang nicht an Versuchen gefehlt, die Eisentröpfchen aus dem Kohlegas auszufiltern. Dies wird jedoch durch die hohe Temperatur des Kohlegases am Austritt aus dem Eisenbadreaktor außergewöhnlich erschwert. Ein erfolgreicher Ansatz zur Entstaubung ist aus der DE-OS 30 41 010 ersichtlich. Derartige Maßnahmen einer Filterung sind notwendig, wenn das erzeugte Kohlegas an öffentliche Gasnetze abgegeben werden soll. Dort ist üblicherweise ein Staubgehalt unter 5 $mg/m^3$ einzuhalten, der Anteil an Eisentropfen im (ungereinigten) Kohlegas erreicht jedoch häufig Werte um 20 bis 30 $g/m^3$. Das ausgefilterte Material wird nach dem Stand der Technik als Abfall angesehen.

Dieses bislang als Abfall angesehene Material eignet sich jedoch überraschenderweise als Katalysator für das Verfahren zur Synthese von Kohlenwasserstoffen entsprechend dem Kölbel-Engelhardt-Verfahren. Die Eisentröpfchen haben aufgrund ihrer äußerst kleinen Abmessungen einen sehr hohen Oberflächenanteil, bieten also als Katalysator ideale Eigenschaften aufgrund ihrer außerordentlich großen, reaktiven Oberfläche. Bei der Verwendung der Eisentröpfchen als Eisenkatalysator für das Synthese-Verfahren müssen die Eisentröpfchen nicht aufbereitet werden, sondern können direkt eingesetzt werden. Insbesondere können sie in der Form, wie sie im erzeugten Kohlegas schweben, unmittelbar einem Syntheseofen zugeführt werden. Somit wird Arbeitszeit, Material und Energie eingespart, weiterhin können die bislang als lästiges Abfallprodukt angesehenen Eisentröpfchen einer sehr hochwertigen Weiterverwendung zugeführt werden. Schließlich ist es nicht mehr notwendig, den Katalysator auf die zur Durchführung des Verfahrens notwendige Temperatur aufzuheizen, vielmehr kann man die in den Eisentröpfchen enthaltene, physikalische Wärme nutzen.

Insbesondere vorteilhaft ist es, das im Eisenbadreaktor erzeugte CO-reiche Gas als Kohlegas für die Synthese einzusetzen. Hierzu wird dieses auf die notwendige Temperatur von etwa 180 bis 280 °C abgekühlt und nach Zugabe von Wasser, insbesondere vermischt mit Wasserdampf, mit den als Katalysatoren dienenden Eisentröpfchen in Berührung gebracht. Dabei kann eine vor dem Syntheseofen erfolgende Trennung der Eisentröpfchen aus dem Kohlegasstrom entfallen, so daß ein einfach ablaufendes Verfahren erreicht wird. Dieses wird weiter dadurch vereinfacht, daß man den Kohlevergasungsvorgang im Eisenbadreaktor bei demjenigen Überdruck durchführt, unter dem auch das Synthese-Verfahren abläuft. Damit können zwischenzuschaltende Reinigungsstufen und Verdichter eingespart werden. Es ist nur noch notwendig, das Kohlegas des Eisenbadreaktors, in dem die Eisentröpfchen schweben, auf die obengenannte, für die Synthese notwendige Temperatur herabzukühlen, wobei die entsprechende Abwärme für andere Prozesse genutzt werden kann. Insgesamt wird also ein erheblicher apparativer Aufwand im Vergleich zur getrennten Verwendung des Kohlegases im Industriebereich oder für Gasnetze bzw. im Vergleich zum getrennten Ablauf der Kölbel-Engelhardt-Synthese gemäß dem Stand der Technik vermieden. Die Kombination von Kohlevergasung im Eisenbadreaktor und Kölbel-Engelhardt-Verfahren spart überraschend viele Zwischenschritte, die bei getrenntem Ablauf beider Verfahren notwendigerweise anfallen, und spart somit Arbeitskraft und Energie.

Es ist jedoch auch möglich, die Eisentropfen zunächst zumindest größtenteils aus dem Kohlegas zu entfernen, insbesondere auszufiltern (s. z. B. DE-OS 30 41 010) und anschließend getrennt vom Kohlegas in den Syntheseofen einzugeben. Hiermit ist eine bessere Steuerung des Verhältnisses von Katalysator zu Kohlegas möglich.

Bei der klassischen Kohlevergasung im Eisenbadreaktor wird die Oberfläche der Eisentröpfchen bereits hinsichtlich ihrer Getterwirkung ausgenutzt. Bekanntlich absorbiert die frische Eisenoberfläche Schwefel, im Gasraum oberhalb des Eisenbadreaktors findet zusätzlich zur Schlacke somit eine Nachentschwefelung des Kohlegases statt, da Schwefel an den Eisentröpfchen adsorbiert wird. Derart mit Schwefel verunreinigte Eisentröpfchen sind jedoch bei dem erfindungsgemäßen Verfahren ungünstig. Es wird daher vorgeschlagen, den Eisenbadreaktor so zu betreiben, daß der Schwefelgehalt des Kohlegases möglichst gering ist.

Vorteilhafterweise werden anschließend an die Synthese im Syntheseofen die Eisentropfen aus der Öl-Kontakt-Suspension oder aus den erzeugten Kohlewasserstoffen entfernt. Hierzu dienen klassische Methoden der Trennung bzw. Filterung. Insbesondere ist es vorteilhaft, die Eisentröpfchen in einer Zentrifuge oder aber magnetisch abzutrennen.

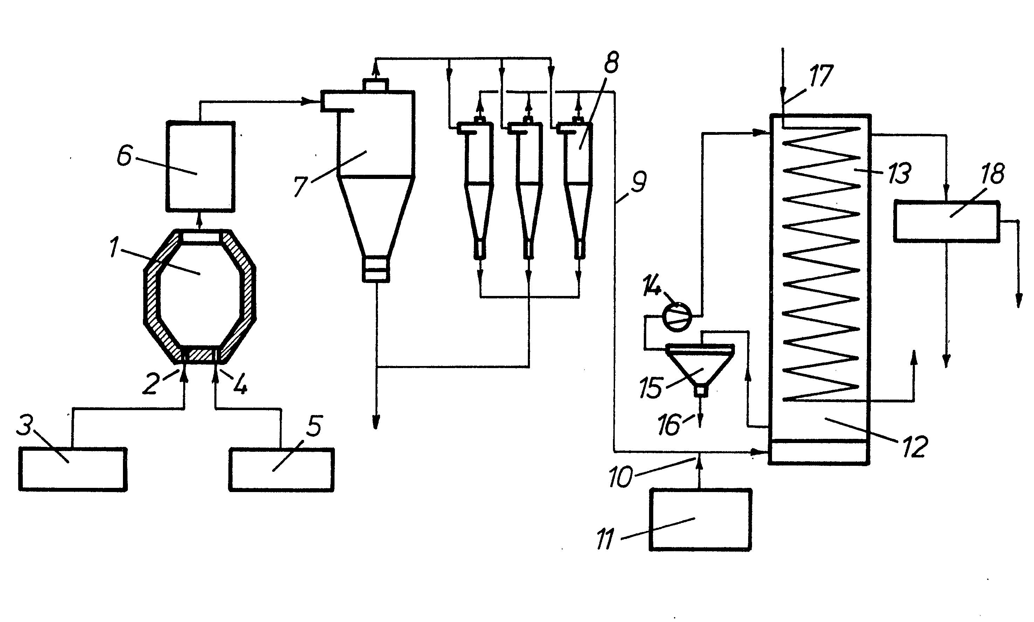

Ein Ausführungsbeispiel des erfindungsgemäßen Verfahrens wird im folgenden näher erläutert und unter Bezugnahme auf die Zeichnung beschrieben, die in ihrer einzigen Figur eine Anlage zur Herstellung benzinartiger Kohlenwasserstoffe nach dem erfindungsgemäßen Verfahren zeigt.

Einem Eisenbadreaktor 1 mit einem Konvertergefäß wird von unten durch eine Bodendüse 2 feinkörnige bzw. staubförmige Kohle aus einem Vorratsbehälter 3 und durch eine Bodendüse 4 bzw. durch obere, nicht dargestellte und auf die Schmelze gerichtete Düsen Sauerstoff aus einem Vorratsbehälter 5 zugeführt. Die Kohlevergasung erfolgt unter einem Druck von 10 bis 12 bar. Das oben aus dem Eisenbadreaktor 1 heraustretende Kohlegas wird in einem Abhitzekessel 6 auf ca. 350 °C abgekühlt. Die dabei erhaltene fühlbare Wärme wird weiterverwendet, beispielsweise zur Dampferzeugung genutzt.

Das abgekühlte Kohlegas wird anschließend durch einen ersten Zyklon 7 und daraufhin durch drei, parallel geschaltete Multi-Zyklone 8 geleitet. Diese befreien das Kohlegas von Grobstaub und sind so eingestellt, daß die im Kohlegas mitgeführte, in feinster Verteilung vorliegenden Eisentröpfchen nicht abgeschieden werden, sondern zusammen mit dem unter Überdruck stehenden Kohlegas über eine Leitung 9, in die bei 10 für die CO-Umsetzung erforderliche Wassermenge aus einem Speicher 11 dampfförmig eingespeist wird, einem Syntheseofen 12 zugeführt werden.

Der Syntheseofen 12 wird in Flüssigphase-Technik betrieben, er ist mit einem Öl 13 gefüllt. Beim Durchgang des mit den Eisentröpfchen und weiterem Feinstaub beladenen Kohlegases durch den Syntheseofen 12 erfolgt die katalytische Umsetzung des Kohlenmonoxides mit dem Wasserstoff bzw. dem zugesetzten Wasser zu Kohlenwasserstoff und $CO_2$. Die Eisentröpfchen und die feinen Schmutzpartikel bleiben im Öl 13 hängen und werden ausgewaschen. Hierzu dient ein Ölkreislauf mit einer Pumpe 14 und einer Zentrifuge 15, an deren unterem Ausgang 16 die Eisentröpfchen und die feinen Schmutzpartikel abgezogen werden. Das Öl 13 wird somit ständig in Umlauf gehalten und gereinigt.

Die im Syntheseofen 12 anfallende Prozeßwärme wird über eine Leitung 17 abgeführt, vorzugsweise wird Dampf erzeugt, dessen Energie für einzelne Prozeßschritte, insbesondere für die

noch zu beschreibende CO$_2$-Wäsche dient.

Die Abtrennung der Kohlenwasserstoffe erfolgt in einer sich an den Syntheseofen 12 anschließenden Aufbereitungsstufe 18. Der im Abhitzekessel 6 sowie bei der exothermen Kohlenwasserstoffsynthese erzeugte Dampf wird als Antriebsenergie für eine Luftzerlegungsanlage sowie als Prozeßdampf in der CO$_2$-Wäsche bzw. bei der Aufbereitung der Rohprodukte eingesetzt.

Die vorgeschlagene Verfahrensweise läßt eine Verbesserung des thermischen Wirkungsgrades gegenüber den herkömmlichen Direkt-Hydrier-Verfahren und der Fischer-Tropsch-Synthese sowie eine Verminderung der Investitions- und Betriebskosten zu, da die Konvertierungsstufe sowie die hieran anschliessende CO$_2$-Wäsche und Entschwefelung mit der hiermit verbundenen Zwischenabkühlung des Gases entfallen und die im Gas enthaltenen Eisentröpfchen die Synthese begünstigen. Es kann bei der Synthese mit einer 90 %-igen Umsetzung der eingebrachten CO- und der H$_2$-Anteile gerechnet werden. Dies entspricht bei der Kombination mit der Kohlevergasung im Eisenbadreaktor einem Anfall von etwa 360 kg flüssiger Kohlenwasserstoffverbindungen ($H_u$ = 10 400 Kcal/kg) pro Tonne eingesetzter Rohkohle ($H_u$ = 6 200 Kcal/kg).

## Ansprüche

1. Verfahren zur Herstellung benzinartiger Kohlenwasserstoffe, bei dem ein Kohlegas, d. h. ein CO- und H$_2$-haltiges Gas, im erforderlichen Mengenverhältnis mit Wasser, insbesondere Wasserdampf gemischt wird und dieses Gemisch über einen Eisen-Katalysator geführt wird, dadurch gekennzeichnet, daß als Eisenkatalysator die bei der Kohlevergasung im Eisenbadreaktor im erzeugten Kohlegas suspendierten, feinstverteilten Eisentropfen verwendet werden.

2. Verfahren nach Anspruch 1, dadurch gekennzeichnet, daß das im Eisenbadreaktor erzeugte, CO-reiche Gas zunächst auf eine für die Synthese günstige Temperatur von 180 bis 380 °C abgekühlt und anschließend nach Zugabe von Wasser, insbesondere nach Vermischen mit Wasserdampf, mit den Eisentropfen in Berührung gebracht wird.

3. Verfahren nach Anspruch 1 und 2, dadurch gekennzeichnet, daß die Synthese in einem Syntheseofen bei einem Überdruck von 15 bar durchgeführt wird und daß die Kohlevergasung im Eisenbadreaktor im wesentlichen bei demselben Überdruck durchgeführt wird, unter dem die Synthese im Syntheseofen abläuft.

4. Verfahren nach einem oder mehreren der Ansprüche 1 bis 3, dadurch gekennzeichnet, daß die Kohlegaserzeugung im Eisenbadreaktor bei Normaldruck abläuft und daß das erzeugte Kohlegas anschließend auf einen für die Synthese günstigen erforderlichen Überdruckwert, insbesondere 15 bar, komprimiert wird.

5. Verfahren nach einem oder mehreren der Ansprüche 1 bis 4, dadurch gekennzeichnet, daß das im Eisenbadreaktor erzeugte Kohlegas zusammen mit den in ihm schwebenden Eisentropfen dem Syntheseofen für die Synthese zugeführt wird.

6. Verfahren nach einem oder mehreren der Ansprüche 1 bis 4, dadurch gekennzeichnet, daß die Eisentropfen zunächst zumindest größtenteils aus dem Kohlegas entfernt, insbesondere ausgefiltert werden und anschließend getrennt vom Kohlegas in den Syntheseofen eingegeben werden.

7. Verfahren nach einem oder mehreren der Ansprüche 1 bis 6, dadurch gekennzeichnet, daß der Eisenbadreaktor so betrieben wird, insbesondere die Schlackenzusammensetzung so gewählt wird, daß der Schwefelgehalt des Kohlegases möglichst gering ist.

8. Verfahren nach einem oder mehreren der Ansprüche 1 bis 7, dadurch gekennzeichnet, daß anschließend an die Synthese im Syntheseofen die Eisenpartikel aus einem Kontaktöl des Syntheseofens oder aus den erzeugten Kohlewasserstoffen ausgeschieden, insbesondere in einer Zentrifuge oder in einem Magnetabscheider abgeschieden werden.

## Claims

1. A process for preparing gasoline-like hydrocarbons, by which a coal gas, i. e. a gas containing CO and H$_2$, is mixed in the necessary proportion with water, in particular water vapor, and this mixture is directed through an iron catalyst, characterized in that the extremely finely distributed iron drops suspended in the generated coal gas in the iron bath reactor during the gasification of coal are used as the iron catalyst.

2. The process as in claim 1, characterized in that the gas rich in CO generated in the iron bath reactor is first cooled down to a temperature of 180 to 380 °C favorable for synthesis, and then put in contact with the iron drops after water is added, in particular after being mixed with water vapor.

3. The process as in claims 1 and 2, characterized in that the synthesis is carried out in a synthesis oven at an overpressure of 15 bar, and in that the gasification of coal is carried out in the iron bath reactor at substantially the same overpressure under which the synthesis takes place in the synthesis oven.

4. The process as in one or more of claims 1 to 3, characterized in that the generation of coal gas takes place in the iron bath reactor at normal pressure and in that the generated coal gas is then compressed to an overpressure value, in particular 15 bar, which is necessary and favorable for the synthesis.

5. The process as in one or more of claims 1 to 4, characterized in that the coal gas generated in the iron bath reactor is fed, together with the iron drops suspended therein, into the synthesis oven for synthesis.

6. The process as in one or more of claims 1 to 4, characterized in that the iron drops are first removed from, in particular filtered out of, the coal gas, at least for the most part, and are then fed into the synthesis oven separately from the coal gas.

7. The process as in one or more of claims 1 to 6, characterized in that the iron bath reactor is operated, in particular the slag composition is selected, in such a way that the sulphur content of the coal gas is as low as possible.

8. The process as in one or more of claims 1 to 7, characterized in that following the synthesis in the synthesis oven the iron particles are separated from a contact oil of the synthesis oven or from the generated hydrocarbons, in particular precipitated in a centrifuge or in a magnetic separator.

**Revendications**

1. Procédé pour la fabrication d'hydrocarbures de type essence dans lequel un gaz de houille, c'est-à-dire un gaz contenant du CO et du $H_2$, est mélangé dans le rapport quantitatif requis avec de l'eau, notamment de la vapeur d'eau, et ce mélange est amené à passer sur un catalyseur au fer, caractérisé en ce que comme catalyseur au fer sont utilisées les gouttelettes de fer très finement divisé se trouvant lors de la gazéification de charbon dans le réacteur à bain de fer en suspension dans le gaz de houille produit.

2. Procédé selon la revendication 1, caractérisé en ce que le gaz riche en CO produit dans le réacteur à bain de fer est d'abord refroidi jusqu'à une température de 180 à 380 °C favorable pour la synthèse et est ensuite après adjonction d'eau, notamment après mélange avec de la vapeur d'eau, amené en contact avec les gouttelettes de fer.

3. Procédé selon la revendication 1 ou 2, caractérisé en ce que la synthèse est réalisée dans un four de synthèse sous une surpression de 15 bars et en ce que la gazéification de charbon se réalise dans le réacteur à bain de fer sensiblement sous la même pression élevée que celle sous laquelle se déroule la synthèse dans le four de synthèse.

4. Procédé selon l'une quelconque des revendications précédentes, caractérisé en ce que la production de gaz de houille dans le réacteur à bain de fer se déroule à la pression ordinaire et en ce que le gaz de houille produit est ensuite comprimé de façon à présenter une valeur de surpression requise, en particulier 15 bars, favorable pour la synthèse.

5. Procédé selon l'une quelconque des revendications précédentes, caractérisé en ce que le gaz de houille produit dans le réacteur à bain de fer est amené, conjointement avec les gouttelettes de fer flottant dans ce gaz, au four de synthèse en vue de la synthèse.

6. Procédé selon l'une quelconque des revendications 1 à 4, caractérisé en ce que les gouttelettes de fer sont d'abord tout au moins en majeure partie éliminées du gaz de houille, en particulier par filtration, et sont ensuite introduites, séparément du gaz de houille, dans le four de synthèse.

7. Procédé selon l'une quelconque des revendications précédentes, caractérisé en ce que le réacteur à bain de fer est amené à fonctionner et en particulier la composition des scories est choisie de telle manière que la teneur en soufre du gaz de houille soit aussi réduite que possible.

8. Procédé selon l'une quelconque des revendications précédentes, caractérisé en ce qu'à la suite de la synthèse réalisée dans le four de synthèse les particules de fer sont séparées d'une huile catalytique du four de synthèse ou des hydrocarbures produits, et ce en particulier dans une centrifugeuse ou dans un séparateur magnétique.